# EUROPEAN PATENT SPECIFICATION

(11) **EP 0 408 005 B1**
(45) Date of publication and mention of the grant of the patent: **02.03.1994**
(21) Application number: 90113264.7
(22) Date of filing: 11.07.1990
(51) Int. Cl.: C07C 19/08, C07C 17/00

(54) **Process for preparing 1,1,1,2-tetrafluoroethane**
Verfahren zur Herstellung von 1,1,1,2-Tetrafluorethan
Procédé pour préparer du 1,1,1,2-tétrafluoroéthane

(30) Priority: 12.07.1989 IT 2115989
(43) Date of publication of application: 16.01.1991
(73) Proprietor: AUSIMONT S.p.A., I-20121 Milano (IT)
(72) Inventor: Cuzzato, Paolo, I-31100 Treviso (IT); Masiero, Antonio, I-35048 Stanghella, Padova (IT)
(74) Representative: Barz, Peter, Dr.

(56) References cited:
- GB-A- 819 849

## Description

The present invention relates to a process for preparing, in the gas phase, 1,1,1,2-tetrafluoroethane by reaction of trichloroethylene (CHCl=CCl₂) with hydrofluoric acid in the presence of catalysts.

It is known that it is possible to obtain 1,1,1,2-tetrafluoroethane (hereafter referred to as 134a) by catalytic reaction in the gas phase of 1,1,1 trifluorochloroethane (hereafter referred to as 133a) and hydrofluoric acid according to the reaction 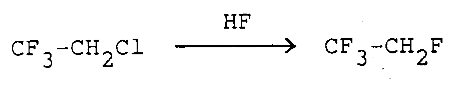
According to US A-4 129 603 said reaction is carried out by using a catalyst consisting of chromium oxide or, at least partially, of basic chromium fluoride, at temperatures ranging from 300° to 400°C, thereby obtaining 133a conversions of 20%, with yields of 134a equal to 91%.

According to said process, also CF₂=CHCl is formed (as a result of the dehydrofluorination of 133a), the boiling point of which (only 9°C higher than the boiling point of 134a) makes the separation thereof difficult and uneconomic.

Therefore, BE-A-863 913 describes a method for reducing or eliminating the above impurity from the reaction products, which method consists of the post-fluorination of the olefin at low temperature and with the same type of catalyst.

According to another method, described in CA-A-1 124 265, said olefin content is reduced to 5 to 10 ppm by contacting the 133a fluorination products with an aqueous solution of KMnO₄.

Other processes for preparing 134a by means of fluorination of 133a with catalysts based on chromium oxide are described in JP-A-80-27138 and in DE-A-2 932 934, according to which applications conversions of 21% and 31%, with 134a yields of 91% and 98%, respectively, are obtained.

EP-A-300 724 describes a process for preparing, in the liquid phase, 134a by fluorination of 133a in the presence of catalysts based on antimony halides. Said process has the drawback of not being highly selective, in particular, owing to the considerable amount of pentafluoroethane produced. In said document, the difficulty of producing 134a through fluorination of trichloroethylene in the gas phase is mentioned.

It has, in fact, been experimentally confirmed that the direct fluorination of trichloroethylene in the gas phase using, for example, a chromium oxyfluoride catalyst, produces 134a with yields of only 3%, although it provides high yields of 133a and trichloroethylene conversions of 92%.

Furthermore, the catalyst activity rapidly decreases, wherefore said process, if it were utilized to produce 134a in acceptable yields, would require frequent reactivation of the catalyst, which is prejudicial to its use on an industrial scale.

An alternative would be to prepare 133a separately by fluorination of trichloroethylene in the liquid phase and then using it for preparing 134a through fluorination in the gas phase.

This solution would, however, result in considerable investment costs since two distinct plants with two different technologies would be required.

It has now, surprisingly, been found that it is possible to provide an industrial process in the gas phase for preparing 134a with industrially acceptable conversions, the process being furthermore highly selective. Said process comprises the reaction of a mixture of trichloroethylene and 133a, in trichloroethylene/133a molar ratios ranging from 5/95 to 50/50, with hydrofluoric acid, in the presence of a catalyst comprising Cr₂O₃ carried on aluminium trifluoride, prepared by impregnating AlF₃ comprising gamma- and/or beta-AlF₃ with a solution of a soluble salt of trivalent chromium, drying, and activating the catalyst with air or nitrogen at 200 to 600°C.

In said process, which is the object of the present invention, the catalyst, surprisingly, retains its full activity for very long periods of time, of the order of hundreds of hours, thereby permitting the realisation of the process on an industrial scale and continuously.

PCT application WO 90/08755, a document relevant under Art. 54(3) and (4) EPC, discloses a similar process, save for the way in which the catalyst is prepared (no impregnation of AlF₃).

A preferred mode of carrying out the present process consists of feeding, at the beginning, the reactor containing the catalyst with the trichloroethylene/133a mixture (in the above ratio) along with hydrofluoric acid, of separating, at the reactor outlet, the formed 134a from the other reaction products (predominantly consisting of 133a) and of recycling said products to the reactor (after addition of trichloroethylene and hydrofluoric acid in order to readjust the above ratio of reagents).

Preferably, the reaction of trichloroethylene, 133a and hydrofluoric acid is conducted at temperatures of from 300 to 400°C, particularly from 330 to 380°C at atmospheric pressure or at higher pressures, up to 1.5 MPa (15 atmospheres).

Preferred feeding conditions are trichloroethylene/133a molar ratios of about 15/85. Generaily, the HF/trichloroethylene + 133a molar ratio is not lower than 3, while particularly critical upper limits for said ratio do not exist. It is, however, preferable to use HF/trichloroethylene + 133a molar ratios of from 3/1 to 10/1 and, even more preferred, from 4/1 to 6/1.

The contact time of reagents and catalyst is not critical, although, generally, the contact time is not lower than one second. Usually, contact times ranging from 1 to 50 seconds and, preferably, from 5 to 20 seconds, are employed.

The present process may be conducted both discontinuously (collecting the reaction products after only one run) and continuously (recycling the unreacted trichloroethylene and the 133a to the reactor, after having readjusted the amount of reagents in the above ratio, as mentioned above).

The catalyst to be used in the process of the present invention is composed of chromium trioxide supported on AlF₃, comprising the gamma and/or beta form.

The amount of Cr₂O₃ generally ranges from 1 to 15% by weight, calculated as Cr, based on the supported catalyst. The optimum percentage of Cr₂O₃ is a function of the surface area of AlF₃ in the gamma form.

Carriers having a high surface area, of the order of 25 to 30 m²/g, are generally preferred.

The carrier can be in the form of a powder having a particle size generally ranging from 20 to 200 µm. If required, it can, however also be in the form of pellets.

Besides the gamma and/or beta form, AlF₃ may also comprise the delta form, generally in amounts of up to 30% by weight.

AlF₃ in the alpha form can also be present, although it is preferred that the amount thereof be limited, since this form has shown relatively low activity.

The catalyst to be employed according to the present invention can is prepared as follows:
The AlF₃ carrier in the above crystallographic forms is impregnated, according to one of the conventional techniques of the art, in wet condition or in dry condition, with a solution of a soluble salt of trivalent chromium, for example, CrCl₃·6H₂O.

The catalyst is then dried in order to remove the water present therein, then it is placed into a reactor and is subjected to an activation treatment with air or nitrogen, in the presence or absence of steam and/or crystallization water which can act as oxidant.

The activation treatment is carried out at temperatures ranging from 200 to 600°C, preferably from 350 to 500°C, in order to convert chromium into the oxide form.

The above allotropic structures of AlF₃ are known and may be characterized by means of their X-ray diffraction spectrum as described, for example, in J.C.P.D.S. 1981 and in FR-A-1 383 927.

The above gamma_{C}, delta_{C} and beta_{C} phases are those which are described in FR-A-1 383 927 by J. Christoph and J. Teufer. The alpha phase is described in Anal. Chem. 29, 984 (1957).

After prolonged use, the catalytic activity can be restored by means of an air treatment at high temperatures (from 350 to 500°C).

The following examples merely serve to illustrate the invention without being a limitation of the scope thereof.

### Example 1

### Catalyst Preparation

A tubular Inconel reactor having a diameter of 8 cm and a length of 100 cm, electrically heated and equipped with a sintered porous Inconel baffle, was charged with 1680 g of a catalyst, prepared as described below.
A carrier consisting of AlF₃, prevailingly in the gamma form and having a specific surface area of 26 m²/g, was impregnated with an aqueous solution of CrCl₃·6H₂O in an amount of 492 g of CrCl₃·6H₂O per kg of AlF₃.

The solution, consisting of 492 g of CrCl₃·6H₂O + 152 ml of H₂O, had a volume of 450 ml and was added to the AlF₃ in three almost equal portions. After each addition, the catalyst was dried for 4 hours at 120°C and at atmospheric pressure.

After the third drying operation, the catalyst was also sieved and placed into the reactor.

The catalyst was fluidized with a nitrogen stream (about 100 l/h) for 10 hours in the reactor heated to 400°C, then the reactor was brought to the operating temperature.

### Example 2 (Comparative Test)

The above reactor was charged, at 380°C with 1.536 moles/h of trichloroethylene and 9.137 moles/h of anhydrous HF, resulting in a HF/C₂HCl₃ molar ratio of 6 and a contact time of 9.6 seconds, calculated as ratio of non-fluidized catalyst volume to volumetric flow rate of the reagents at the reaction temperature and pressure (the pressure was slightly higher than atmospheric pressure).

The gases leaving the reactor were collected for 1 hour. After absorption of HCl and HF in water and washing of the reaction product with an aqueous NaOH solution, 182 g of a product were recovered, the molar composition of which was as follows:

| | |
|---|---|
| CF₃CH₂Cl | 87.9% |
| CF₃CH₂F | 2.0% |
| C₂HCl₃ | 4.9% |

The balance was predominantly composed of CF₃CHF₂ and CF₃CH₃. The conversion of C₂HCl₃ was 95.1% and the selectivity in CF₃CH₂Cl was 92.4%, while the selectivity in CF₃CH₂F was 2.1%. These results were obtained, without any variations, for about 50 hours of operation, whereafter the catalyst activity began to decrease.

### Example 3

The above reactor was charged, at 350°C and a slightly higher pressure than atmospheric pressure, with 0.072 moles/h of C₂HCl₃, 0.62 moles/h of CF₃CH₂Cl and 3.814 moles/h of anhydrous HF, thus obtaining a contact time of 19.5 seconds, a HF/organic product molar ratio of 5.5 and a percentage of C₂HCl₃ in the total organic product of 10.4%.

Operating as in example 2, 80 g of a product were recovered, the molar composition of which was as follows:

| | |
|---|---|
| CF₃CH₂Cl | 82.1% |
| CF₃CH₂F | 16.5% |

The balance consisted of small amounts of CF₂=CHCl, CHCl=CCl₂, CF₃CHF₂ and CF₃CH₃. The trichloroethylene conversion was almost quantitative, the total conversion was 17.7% and the selectivity in CF₃CH₂F was equal to 93.2%.

### Example 4

Into the above reactor the following were introduced under the conditions of example 3: 0.194 moles/h of C₂HCl₃, 1.010 moles/h of CF₃CH₂Cl and 7.403 moles/h of anhydrous HF, resulting in a contact time of 10.2 seconds, a HF/organic product ratio of 6.2 and a percentage of C₂HCl₃ in the total organic product of 16.1%.

Operating in similar manner as in the preceding example, 140 g of a product were obtained, the molar composition of which was as follows.

| | |
|---|---|
| CF₃CH₂Cl | 85.0% |
| CF₃CH₂F | 13.6%. |

The by-products were the same as in example 3.

The trichloroethylene conversion was almost quantitative, the total conversion was equal to 14.5% and the selectivity in CF₃CH₂F was 93.8%.

### Example 5

Into the above reactor and under the conditions of example 3, 0.250 moles/h of C₂HCl₃, 0.714 moles/h of CF₃CH₂Cl and 7.468 moles/h of anhydrous HF were introduced, thereby obtaining a contact time of 10.4 seconds, a HF/organic product ratio equal to 7.8 and a percentage of C₂HCl₃ in the total organic product of 25.9%.

Operating in similar manner as in the preceding examples, 112 g of a product of the following molar composition were obtained:

| | |
|---|---|
| CF₃CH₂Cl | 86.9% |
| CF₃CH₂F | 11.8% |

The by-products were the same as in example 3.

The trichloroethylene conversion was almost quantitative, the total conversion was 12.9% and the selectivity in CF₃CH₂F was 91.5%.

Under these conditions, as well as under those of the preceding examples, the catalyst, after about 200 hours of operation, did not show any significant decrease in activity.

### Example 6

The reactor of the preceding examples was converted into a plant capable of operating continuously, by addition of a separation column by which the light products, including 134a (CF₃CFH₂) were withdrawn, while CF₃CH₂Cl and the higher-boiling products were conveyed to a pump and fed again to the reactor, along with fresh trichloroethylene and HF, in order to make up for the consumptions.

The amount of fresh make-up products was slightly varied over the length of time in order to maintain constant the trichloroethylene/CF₃CH₂Cl and HF/organic product ratios. The reference conditions were:
Reaction temperature = 350°C; Contact time = 10 seconds; HF/organic product ratio = 6; trichloroethylene/CF₃CH₂Cl ratio = 15/85 at the reactor inlet.

After the plant had been adjusted to the operating conditions with a trichloroethylene/CF₃CH₂Cl mixture prepared in advance, there were introduced, over a period of 6 hours, 1.08 moles of fresh trichloroethylene and 4.50 moles of fresh anhydrous HF, while about 1 mole of CF₃CH₂F and small amounts of by-products of the same nature as those mentioned in the preceding examples were withdrawn from the top of the distillation column.

## Claims

1. Process for preparing 1,1,1,2-tetrafluoroethane, which comprises reacting, in the gas phase, trichloroethylene with 1,1,1-trifluorochloroethane and hydrofluoric acid, in molar ratios of trichloroethylene to 1,1,1-trifluorochloroethane of from 5/95 to 50/50 and in the presence of a catalyst comprising chromium trioxide supported on aluminium trifluoride, prepared by impregnating AlF₃ comprising gamma- and/or beta-AlF₃ with a solution of a soluble salt of trivalent chromium, drying, and activating the catalyst with air or nitrogen at 200 to 600°C.

2. Process according to claim 1, wherein the reaction is carried out at a temperature of from 300 to 400°C.

3. Process according to claim 1, wherein the reaction is carried out at a temperature of from 330 to 380°C.

4. Process according to any one of claims 1 to 3, wherein the contact time of the reagents ranges from 1 to 50 seconds.

5. Process according to any one of claims 1 to 4, wherein the contact time of the reagents ranges from 5 to 20 seconds.

6. Process according to any one of claims 1 to 5, wherein the aluminium trifluoride exhibits a specific surface area of from 25 to 30 m²/g.

7. Process according to any one of claims 1 to 6, which is carried out continuously.

## Patentansprüche

1. Verfahren zur Herstellung von 1,1,1,2-Tetrafluorethan, umfassend die Umsetzung, in der Gasphase, von Trichlorethylen mit 1,1,1-Trifluorchlorethan und Fluorwasserstoffsäure, in Molverhältnissen von Trichlorethylen zu 1,1,1-Trifluorchlorethan von 5/95 bis 5/50 und in Anwesenheit eines auf Aluminiumtrifluorid getragenes Chromtrioxid umfassenden Katalysators, hergestellt durch Imprägnierung von AlF₃, das gamma- und/oder beta-AlF₃ umfaßt, mit einer Lösung eines löslichen Salzes von dreiwertigem Chrom, Trocknung und Aktivierung des Katalysators mit Luft oder Stickstoff bei 200 bis 600°C.

2. Verfahren nach Anspruch 1, in welchem die Umsetzung bei einer Temperatur von 300 bis 400°C durchgeführt wird.

3. Verfahren nach Anspruch 1, in welchem die Umsetzung bei einer Temperatur von 330 bis 380°C durchgeführt wird.

4. Verfahren nach irgendeinem der Ansprüche 1 bis 3, in welchem die Kontaktzeit der Reagenzien im Bereich von 1 bis 50 Sekunden liegt.

5. Verfahren nach irgendeinem der Ansprüche 1 bis 4, in welchem die Kontaktzeit der Reagenzien im Bereich von 5 bis 20 Sekunden liegt.

6. Verfahren nach irgendeinem der Ansprüche 1 bis 5, in welchem das Aluminiumtrifluorid eine spezifische Oberfläche von 25 bis 30 m²/g zeigt.

7. Verfahren nach irgendeinem der Ansprüche 1 bis 6, welches kontinuierlich durchgeführt wird.

## Revendications

1. Procédé de préparation de 1,1,1,2-tétrafluoroéthane consistant à faire réagir, en phase gazeuse, du trichloroéthylène avec du 1,1,1-trifluorochloroéthane et de l'acide fluorhydrique, dans des rapports molaires trichloroéthylène/1,1,1-trifluorochloroéthane compris entre 5/95 et 50/50, et en présence d'un catalyseur composé de trioxyde de chrome mis sur un support de trifluorure d'aluminium, préparé par imprégnation de AlF₃ comprenant les phases gamma et/ou béta à l'aide d'une solution d'un sel soluble de chrome trivalent, séchage et activation du catalyseur à l'air ou à l'azote à une température comprise entre 200 et 600°C.

2. Procédé selon la revendication 1, dans lequel la réaction est effectuée à une température comprise entre 300 et 400°C.

3. Procédé selon la revendication 1, dans lequel la reaction est effectuée à une température comprise entre 330 et 380°C.

4. Procédé selon l'une quelconque des revendications 1 à 3, dans lequel le temps de contact des réactifs est compris entre 1 et 50 secondes.

5. Procédé selon l'une quelconque des revendications 1 à 4, dans lequel le temps de contact des réactifs est compris entre 5 et 20 secondes.

6. Procédé selon l'une quelconque des revendications 1 à 5, dans lequel le trifluorure d'aluminium présente une aire surfacique spécifique de 25 à 30 m²/g.

7. Procédé selon l'une quelconque des revendications 1 à 6, caractérisé en ce qu'il est effectué de manière continue.
